(19) Europäisches Patentamt
European Patent Office
Office européen des brevets

(11) Veröffentlichungsnummer : **0 079 899**
**B1**

(12) # EUROPÄISCHE PATENTSCHRIFT

(45) Veröffentlichungstag der Patentschrift : **05.03.86**

(21) Anmeldenummer : **82901512.2**

(22) Anmeldetag : **21.05.82**

(86) Internationale Anmeldenummer : **PCT/EP 82/00111**

(87) Internationale Veröffentlichungsnummer : **WO/8204391 (23.12.82 Gazette 82/30)**

(51) Int. Cl.⁴ : **A 61 K 7/06**, A 61 K 7/48, C 07 C149/40, C 07 C149/41, C 07 D213/89, C 07 D263/58

(54) **TOPISCHE KOSMETISCHE ZUBEREITUNGEN, ENTHALTEND HETEROARYLMERCAPTO-ALKANSÄURE-DERIVATE ALS ANTISEBORRHOISCHE ZUSÄTZE.**

(30) Priorität : **27.05.81 DE 3121072**

(43) Veröffentlichungstag der Anmeldung : **01.06.83 Patentblatt 83/22**

(45) Bekanntmachung des Hinweises auf die Patenterteilung : **05.03.86 Patentblatt 86/10**

(84) Benannte Vertragsstaaten : **AT BE CH DE FR GB LI LU NL SE**

(56) Entgegenhaltungen :
**EP-A- 0 006 347**
**EP-A- 0 039 857**
**DE-A- 1 667 902**
**FR-A- 2 126 996**
**GB-A- 718 322**
**GB-A- 1 317 773**
**US-A- 3 392 194**
**US-A- 4 012 523**
**US-A- 4 049 665**
**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **MÖLLER, Hinrich**
**Schumannstrasse 11**
**D-4019 Monheim (DE)**
Erfinder : **WALLAT, Siegfried**
**Marie Curie-Strasse 9**
**D-4019 Monheim (DE)**

**EP 0 079 899 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind topische, kosmetische Zubereitungen zur Verbesserung des fettigen und unästhetischen Aussehens der Haare und der Haut, insbesondere zur Behandlung von stark fettendem Haar.

Die übermäßig starke Absonderung der Talgdrüsen der Kopfhaut verleiht dem Haar ein fettiges Aussehen, das im allgemeinen als wenig ästhetisch angesehen wird. Es ist daher vielfach versucht worden, durch geeignete Mittel die Sekretion der Talgdrüsen zu normalisieren, um dem Haar wieder sein gesundes Aussehen zu geben.

Zur Therapie der Seborrhoe wurden bereits Shampoos mit Schwefel, Quecksilber oder Teerzusatz verwendet. Dabei hat sich gezeigt, daß diese Mittel bei längerer Anwendung häufig zu Nebenwirkungen führten, ohne daß wirklich befriedigende Ergebnisse im Hinblick auf Wirksamkeit oder anwendungstechnische Eigenschaften erzielt werden konnten. In der DE-A-1 906 665 werden zur Behandlung der durch Seborrhoe verursachten Schuppenbildung N,N-Diethyl-m-Toluamid, in der US-A-3 755 604 Phenylpentadiensäuren als Mittel zur Verhinderung der Sebumproduktion vorgeschlagen. Es hat sich jedoch gezeigt, daß weder N,N-Diethyl-m-Toluamid noch Phenylpentadiensäuren zufriedenstellende antiseborrhoische Wirkungen zeigen.

Weiterhin ist es aus US-A-3 392 194 bekannt, pharmazeutische, zur topischen Anwendung geeignete Zusammensetzungen, die mindestens einen Wirkstoff der allgemeinen Formel $X—A—CR_1R_2—CONR_3R_4$, (wobei $A = S$, $R_1 = R_2 = CH_3$, $R_3$ und $R_4 = H$, Alkyl, gegebenenfalls substituiertes Aryl, und $X =$ gegebenenfalls substituiertes Aryl sein kann), zur Verminderung der Konzentration des Cholesterins im Blutserum einzusetzen.

In EP-A-6347 sind substituierte Benzazolylthioalkansäureester als Pflanzenwachstumsregulatoren beschrieben. In DE-A-1 667 902 werden peroral zu verabreichende Zubereitungen zur Bekämpfung der Seborrhoe vorgeschlagen, die mindestens eine wirksame Komponente der Formel $R_3—S—C(R_1R_2)—(CH_2)_n—CH_2—NH_2$ enthalten, worin $R_3$ auch ein N-Oxidopyridylrest sein kann. Keine der genannten Veröffentlichungen legt die Erfindung nahe.

Die ältere Patentanmeldung EP-A-39 857 beschreibt Hautpflegemittel mit einem Gehalt an Verbindungen der allgemeinen Formel $Ar—(X)_p—(CR_1R_2)_m—(CR_3R_4)_n—COR_5$, worin Ar für einen gegebenenfalls substituierten Arylrest steht. Eine Überschneidung mit dem Gegenstand der Erfindung liegt somit nicht vor.

Aus FR-A-2 126 996 sind kosmetische Kompositionen bekannt, die als aktive Komponenten Thioglycolat-Derivate mit an S gebundener substituierter Pyridyl-N-Oxidgruppe enthalten. Es hat sich jedoch gezeigt, daß die sebosuppressive Wirksamkeit durch die Einführung eines Substituenten am Pyridyl-N-Oxidring erheblich herabgesetzt wird.

Gegenstand der Erfindung sind topische, kosmetische Zubereitungen, enthaltend Heteroarylmercapto-alkansäure-Derivate der allgemeinen Formel

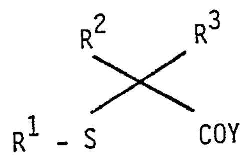

(I)

in der $R^1$ für 2-, 3-, 4-Pyridyl, N-Oxido-2-pyridyl und gegebenenfalls substituiertes Benzoxazol-2-yl, $R^2$ und $R^3$ unabhängig voneinander für Wasserstoff oder eine niedere Alkylgruppe mit 1-4 Kohlenstoffatomen und Y für eine Alkoxygruppe mit 1-12 Kohlenstoffatomen stehen, als antiseborrhoischen Wirkstoff neben üblichen Träger- und Hilfsstoffen.

Die Verbindungen gemäß Formel (I) sind z. T. literaturbekannt und im Handel erhältlich. Bei ihrer Herstellung geht man zweckmäßig von den entsprechenden freien Carbonsäuren aus und setzt diese in an sich bekannter Weise mit Alkoholen zu den Estern um. Die Synthese kann auch durch Alkylierung der entsprechenden Heteroarylmercapto-Verbindungen mit geeigneten $\alpha$-Halogenalkansäure-Derivaten erfolgen.

Als Ausgangsprodukt geeignete Heteroarylmercapto-alkansäuren sind zum Beispiel :

Benzoxazol-2-yl, 5-Chlor-benzoxazol-2-yl-, 5,7-Dichlor-benzoxazol-2-yl, 2-, 3-, 4-Pyridyl-, N-Oxido-2-pyridyl-mercapto-essigsäure, -propionsäure, -buttersäure, -isobuttersäure und -2-ethyl-buttersäure.

Als Alkoholkomponente für die Herstellung der entsprechenden Ester sind zum Beispiel geeignet :

Methanol, Ethanol, Propanol, Isopropanol, n-Butanol, Isobutanol, sek.-Butanol, tert.-Butanol, Pentanol, Hexanol, Octanol, 2-Ethylhexanol, Decanol, Benzylalkohol.

Die erfindungsgemäßen kosmetischen Mittel stellen Lösungen der einzusetzenden wirksamen Verbindungen der Formel (I) in Wasser, in Alkohol, in wäßrig-alkoholischen Mischungen, in Öl, Suspensionen, Gelen, Emulsionen, Salben, Pasten oder Aerosolen dar. Die antiseborrhoischen Arencarbonsäureester können in fast alle zur Behandlung von Haut und Haaren üblichen kosmetischen Mittel eingearbeitet werden, wie zum Beispiel in Haarwässer, Haarshampoos, Haarkuren, Haarspülungen,

oder auch in Hautlotionen und Schüttelmixturen. Neben den Verbindungen der Formel (I) enthalten die erfindungsgemäßen Zubereitungen bekannte Träger- und Hilfsstoffe wie Wasser, organische Lösungsmittel, oberflächenaktive Verbindungen, Öle und Fette, Wachse, Parfümole, Farbstoffe, Konservierungsmittel und dergleichen. Eine vorteilhafte Anwendungsform zur Behandlung von stark fettendem Haar ist das Shampoo. Derartige Shampoos können neben dem sebosuppressiven Wirkstoff anionische, kationische, nichtionische oder amphotere Tenside, Farbstoffe, Duftstoffe, Verdickungsmittel oder Konditionierungsmittel enthalten.

Die erfindungsgemäßen kosmetischen Zubereitungen enthalten die Arencarbonsäure-Derivate in einer Menge von 0,01 bis 20 Gewichtsprozent, vorzugsweise 1 bis 10 Gewichtsprozent bezogen auf das Gewicht der gesamten Zubereitung. Die erfindungsgemäßen Mittel können täglich angewendet werden ; es werden jedoch bereits bei einmaliger wöchentlicher Anwendung zufriedenstellende Ergebnisse erzielt. Die individuelle Dosis, die bei jeder Behandlung angewendet werden sollte, ist nicht kritisch. Nachteilige Nebeneffekte wurden nicht beobachtet. Das fettige Aussehen des Haares wird vermindert und das Nachfetten verzögert, wodurch die Möglichkeit einer normalen Haarpflege gegeben ist. Bei Einsatz der erfindungsgemäßen Mittel in Form von Hautcremes oder Milchpräparaten oder Schüttelmixturen gelingt es, durch regelmäßige Anwendung auf der Haut deren Aussehen dauerhaft zu verbessern.

Die nachfolgenden Beispiele sollen den Gegenstand der Erfindung näher erläutern.

## Beispiele

A) Herstellung von Heteroarylmercapto-alkansäure-Derivaten

1. 5-Chlor-benzoxazol-2-yl-mercaptoessigsäureethylester

In die Natriumethylatlösung aus 250 ml Ethanol und 7,5 g (0,326 Mol) Natrium wurden 60 g (0,323 Mol) 5-Chlor-2-mercapto-benzoxazol gegeben und unter Rühren 59,4 g (0,356 Mol) Bromessigsäureethylester getropft. Nach 2-stündigem Sieden, Filtrieren und Abkühlen fielen 27 g 5-Chlor-benzoxazol-2-yl-mercaptoessigsäure-ethylester vom Fp 69 bis 72 °C aus. Durch Eindampfen der Mutterlauge und Umkristallisieren aus n-Hexan/Aktiv-Kohle wurden noch weitere 28 g (zusammen 63 % d. Th.) Ester vom Pf 67-69 °C erhalten. Analog wurden die folgenden Verbindungen erhalten :

2. 2-Pyridylmercapto-essigsäure-ethylester
Kp. 92 °C/0,07 mbar, $n_D^{20}$ : 1, 554 7

3. 4-Pyridylmercapto-essigsäure-ethylester
Kp. 108 °C/0,22 mbar, $n_D^{20}$ : 1,554 4

4. 2-(2-Pyridylmercapto)-isobuttersäure-ethylester-N-oxid
Kp. 141 °C/0,04 mbar, $n_D^{20}$ : 1,574 0

Die antiseborrische Wirkung der in den erfindungsgemäßen kosmetischen Zubereitungen eingesetzten Verbindungen wurde durch nachfolgend beschriebene Tierversuche näher untersucht. Als Versuchstiere dienten männliche Wistar-Ratten von 220-230 g Körpergewicht. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung beziehungsweise einem Lipidlösungsmittel und auch männliche Ratten, die systemisch mit Östrogen behandelt wurden, nur die normale helle, rosafarbene Haut nach dem Scheren aufweisen ; parallel dazu sind aus den abgeschnittenen Haaren nur noch vergleichsweise sehr geringe Lipidmengen zu extrahieren.

Zur Beurteilung der sebosuppressiven Wirksamkeit wurden die Prüfsubstanzen in Form einer 1 %igen Lösung in Ethanol oder Ethanol/Aceton (1 : 1) jeweils 6 Ratten halbseitig auf das Rückenfell gepinselt. Die andere Seite wurde nur mit dem Lösungsmittel ohne Wirksubstanz behandelt (Kontrollseite).

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unabhängig unter Doppelblindbedingungen visuell abgemustert.

Als 1. Kriterium wurde bewertet, ob die Mehrheit der Beurteiler richtig die behandelte Seite erkannt haben, wobei wie folgt differenziert wurde :

| Zeichen | Anteil der Beurteiler, die eine Wirkung erkannten |
|---|---|
| ++ | 100 % |
| + | > 50 %, 100 % |
| 0 | ≤ 50 % |

Als 2. Kriterium wurde der Unterschied zwischen rechter und linker Seite gewertet, wobei pro Beurteiler und Tier jeweils 1 Punkt zu vergeben war, und zwar in der Weise, daß die dunklere Seite mit 1 und die hellere mit 0 und bei Gleichheit beide Seiten mit 0,5 benotet wurden.

Als 3. Kriterium wurden außerdem noch die Intensitätsunterschiede der Brauntöne nach folgender Skala bewertet :

3 Punkte stark braun
2 Punkte mittel braun
1 Punkt schwach braun
0 Punkte keine Braunfärbung

Signifikante Differenzen zwischen unbehandelter und behandelter Seite nach der zweiten Bewertungsmethode zeigen die lokale Wirksamkeit einer Substanz an. Nach der dritten Bewertungsmethode werden die Punktsummendifferenzen zwischen den unbehandelten Kontrolltieren und jeweils den behandelten und unbehandelten Seiten der Versuchstiergruppe gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet.

| Zeichen | Punktdifferenz |
|---|---|
| ++ | sehr groß ( $>$ 99,9 % Wahrscheinlichkeit) |
| + | signifikant ( $\geq$ 95 % Wahrscheinlichkeit) |
| (+) | deutlich aber $<$ 95 % Wahrscheinlichkeit) |

Die nachfolgende Tabelle zeigt die Bewertungsergebnisse nach vorgenanntem Schema für die untersuchten Substanzen,

Bewertung der sebosuppressiven Effekte

| Substanz gemäß Beispiel | Bewertungsmethode 1 | 2 | 3 |
|---|---|---|---|
| 1 | ++ | ++ | ++ |
| 2 | + | + | ++ |
| 3 | + | ++ | ++ |
| 4 | + | ++ | ++ |
| N,N-Diethyl-m-toluamid (DE-OS 19 o6 665) | + | (+) | (+)-0 |
| S-Carboxymethyl-cystein (DE-OS 16 67 9o3) | 0 | 0 | 0 |

B) Beispiele für erfindungsgemäße topische Mittel zur Behandlung von stark fettendem Haar und seborrhoischer Haut.

| Haarkur | Gewichtsteile |
|---|---|
| Tegin M(R) (Glycerinmono- und -distearat) | 0,7 |
| kationisches Tensid | 2,0 |
| Cholesterin | 0,2 |
| Sojalecithin | 0,3 |
| Emulgade A(R) (Gemisch von Cetylstearylalkohol mit nichtionogenen Emulgatoren) | 8,0 |
| Parfümöl | 0,3 |
| Verbindung gemäß Beispiel 1 | 7,0 |
| Wasser, vollentsalzt | 81,5 |

| Hautcreme | |
|---|---|
| Selbstemulgierendes Gemisch aus Mono/Diglyceriden höherer gesättigter Fettsäuren mit Kaliumstearat (R) Dehydag | 16,0 |
| Cetylstearylakohol mit ca. 12 Mol Ethylenoxid | 1,0 |
| 2-Octyldodecanol | 6,0 |
| Isopropylmyristat | 4,0 |
| Glycerin | 6,0 |
| Verbindung gemäß Beispiel 1 | 7,0 |
| Wasser | 60,0 |

**Patentansprüche**

1. Topische kosmetische Zubereitungen, enthaltend Heteroarylmercapto-alkansäure-Derivate der allgemeinen Formel

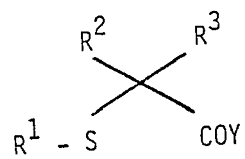

(I)

in der $R^1$ für 2-, 3-, 4-Pyridyl, N-Oxido-2-pyridyl und gegebenenfalls substituiertes Benzoxazol-2-yl, $R^2$ und $R^3$ unabhängig voneinander für Wasserstoff oder eine niedere Alkylgruppe mit 1-4 Kohlenstoffatomen und Y für eine Alkoxygruppe mit 1-12 Kohlenstoffatomen stehen, als antiseborrhoischen Wirkstoff neben üblichen Träger- und Hilfsstoffen.

2. Topische kosmetische Zubereitungen nach Anspruch 1, dadurch gekennzeichnet, daß sie eine Verbindung der allgemeinen Formel (I) in einer Menge von 0,01-20 Gewichtsprozent, vorzugsweise 1-10 Gewichtsprozent, bezogen auf die gesamte Zubereitung, enthalten.

3. 5-Chlor-benzoxazol-2-yl-mercaptoessigsäureethylester.

**Claims**

1. Topical cosmetic preparations containing heteroarylmercapto-alkane acid derivatives corresponding to the following general formula

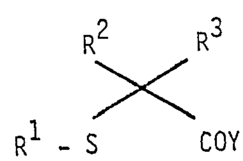

(I)

in which $R^1$ represents 2-, 3-, 4-pyridyl, N-oxido-2-pyridyl and optionally substituted benzoxazol-2-yl, $R^2$ and $R^3$ independently of one another represent hydrogen or a lower $C_1$-$C_4$ alkyl group and Y represents a $C_1$-$C_{12}$ alkoxy group, as antiseborrheic agent in addition to standard carriers and auxiliaries.

2. Topical cosmetic preparations as claimed in Claim 1, characterized in that they contain a compound corresponding to general formula (I) in a quantity of from 0.01 to 20 % by weight and preferably in a quantity of from 1 to 10 % by weight, based on the preparation as a whole.

3. 5-Chlorobenzoxazol-2-yl mercaptoacetic acid ethyl ester.

**Revendications**

1. Préparations cosmétiques topiques, contenant des dérivés d'acides hétéroarylmercapto-alcanoïques de formule générale :

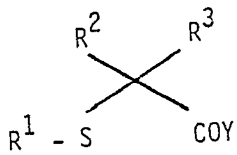

(I)

dans laquelle $R^1$ représente 2-, 3-, 4-pyridyle, N-oxydo-2-pyridyle et benzoxazol-2-yle éventuellement substitué, $R^2$ et $R^3$ indépendamment l'un de l'autre représentent de l'hydrogène ou un groupe alcoyle inférieur ayant 1 à 4 atomes de carbone et Y un groupe alcoxy ayant 1 à 12 atomes de carbone, en tant que matière active anti-séborrhéique à côté de substances de support et auxiliaires usuelles.

2. Préparations cosmétiques topiques selon la revendication 1, caractérisées en ce qu'elles contiennent un composé de formule générale (I) en une quantité de 0,01 à 20 % en poids, de préférence de 1 à 10 % en poids par rapport à la préparation totale.

3. 5-chloro-benzoazol-2-yl-mercaptoacétate d'éthyle.